# EUROPEAN PATENT APPLICATION

(11) **EP 1 942 100 A1**
(43) Date of publication of application: **09.07.2008**
(21) Application number: 07100121.8
(22) Date of filing: 04.01.2007
(51) Int. Cl.: C07C 271/44, A61K 31/27, A61P 25/28

(54) **Amorphous and crystalline forms of rivastigmine hydrogentartrate**

(71) Applicant: Krka Tovarna Zdravil, D.D., Novo Mesto, 8501 Novo Mesto (SI)
(72) Inventor: Benkic, Primoz, 1236 Trzin (SI); Smrkolj, Matej, 1411 Izlake, Zagorje ob Savi (SI); Pecavar, Anica, 8000 Novo mesto (SI); Stropnik, Tadej, 1000 Ljubljana (SI); Vrbinc, Miha, 8000 Novo mesto (SI); Vrecer, Franc, 8351 Straza pri Novem mestu (SI); Pelko Mitja, 8000 Novo mesto (SI)
(74) Representative: UEXKÜLL & STOLBERG

(57) **Abstract**

Rivastigmine hydrogentartrate in amorphous form or in a crystalline form characterized by an X-ray powder diffraction pattern exhibiting peaks at 2Θ values of 5.1, 14.7, 16.5, 17.6, 18.6, 20.4, 21.1° ± 0.2° (Form I), or 2Θ values of 9.5, 11.3, 13.2, 14.1, 15.5, 19.1 20.0° ± 0.2° (Form II), methods for its preparation and pharmaceutical compositions comprising the same.

## Description

### Field of the invention

The present invention relates to novel polymorphic forms of rivastigmine tartrate, as well as to processes for their preparation and pharmaceutical formulations containing the same.

### Background of the invention

Rivastigmine is a non-competitive acetylcholinesterase inhibitor of the carbamate type. It has been shown to inhibit central and peripheral acetylcholinesterases and butyrylcholinesterases and it is indicated for symptomatic treatment of patients with mild to moderately severe Alzheimer's dementia.

Rivastigmine is the INN name for (S)-*N*-ethyl-3-[(1-dimethylamino)ethyl]-*N*-methylphenylcarbamate. It is mostly used in the form of its hydrogen-(2R,3R)-tartrate of formula I

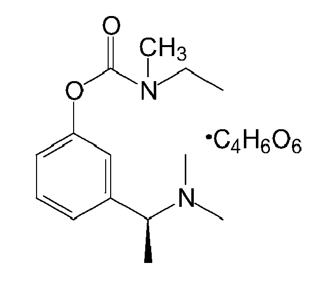

The rivastigmine base is chiral, containing one optical active center and is used as a single stereoisomer (the S-isomer). The (R,R)-hydrogentartrate salt has an overall positive rotation while the free base exhibits a negative optical rotation.

Phenyl carbamates having anticholinesterase activity, *inter alia* rivastigmine, are described in EP 0 193 926 (Proterra). These compounds are prepared in racemic form by reaction of α-m-hydroxyphenylethyldimethylamine with appropriate carbamoyl chlorides. The hydrochlorides are formed by dissolving the free base in ether followed by the introduction of HCl gas; the hydrochloride of rivastigmine is reported to have a melting point of 135-136 °C.

GB 2 203 040 (Sandoz) describes a process for the production of (S)-N-ethyl-N-methyl-3-[1-(dimethylamino)ethyl]-phenyl carbamate and its acid addition salts. The process comprises the separation of the enantiomers from the corresponding racemate by repeated crystallization of the di-0,0'-p-toluoyl tartaric acid salt. The hydrogen tartrate of the (S)-enantiomer (from ethanol) is said to melt at 123-125 °C.

WO 03/101917 (Sun Pharmaceutical) discloses a process for the preparation of phenylcarbamates such as rivastigmine in racemic and enantiomerically active form. Optical resolution is achieved via the di-p-toluoyl tartrate salt. The preparation of the hydrogentartrate salt of rivastigmine is not described.

WO 2004/037771 (Zentiva) concerns the synthesis of rivastigmine and its hydrogentartrate in optically pure form. The hydrogentartrate salt is crystallized from a mixture of ethanol and ethyl acetate by dissolving rivastigmine base and L-(+)-tartaric acid in ethanol at 60 to 70°C and gradually adding ethyl acetate. The resulting mixture is left to crystallize at 5°C. The product is said to have a melting point of 125 to 126°C. Alternatively, the free base and L-(+)-tartaric acid are dissolved in methanol, the clear solution is cooled to room temperature, gradually precipitated with acetone and left to crystallize at 5°C giving a product melting at 123 to 125°C.

WO 2005/058804 (Avecia) discloses the preparation of rivastigmine base in the form of a reddish oil.

WO 2006/068386 (Dong Kook Pharm) discloses a method for synthesizing rivastigmine in a high enantiomeric excess. The (S)-rivastigmine tartrate salt is prepared by reacting rivastigmine base and L-tartaric acid in acetone at reflux temperature. The product precipitates upon cooling to 0°C; the melting point is 124°C.

CN 1486973 (Sanowei) discloses the synthesis of ethyl methyl 3[(1S)-1-(dimethylamino)ethyl] phenyl carbamates by esterification of optically active(S)-3-(1-dimethylamino ethyl) phenol with ethyl methylamino formyl chloride to produce an optically active ester which is then reacted with various salts.

No amorphous or polymorphic forms of rivastigmine hydrogentartrate are reported in the prior art.

### Description of the Invention

It is an object of the present invention to provide rivastigmine hydrogentartrate having a uniform, well-defined morphology. It is a further object of the invention to provide crystal forms of rivastigmine hydrogentartrate having a favorable dissolution rate, bioavailability, inter-individual variability, and chemical stability. It is another object of the present invention to provide rivastigmine hydrogentartrate which exhibits advantageous physical properties, thereby improving the pharmacological properties of the drug and/or its processability when formulated to pharmaceutical dosage forms, such as tablets, capsules, pellets, granules, and powders. The rivastigmine hydrogentartrate should have a uniform morphology meeting the above requirements and should be accessible in a reproducible manner on an industrial scale. Still a further object of the present invention is the provision of a process for the preparation of such forms of rivastigmine hydrogentartrate.

It was surprisingly found that polymorphic forms of rivastigmine hydrogentartrate exist. The present invention provides two novel crystalline forms of rivastigmine hydrogentartrate, rivastigmine hydrogentartrate of polymorphic form (I) and of polymorphic form (II), as well as a process for preparing the same. It was also found that these polymorphic forms can be converted to a stable amorphous form.

Rivastigmine hydrogentartrate of polymorphic form (I) is characterized by the following significant peaks in its X-ray powder diffraction pattern at 2Θ: 5.1, 14.7, 16.5, 17.6, 18.6, 20.4, 21.1° ± 0.2°.

The X-ray powder diffraction pattern of Form (I) comprises the following peaks:

**Table 1**

| No. | Diffraction angles [°; 2Θ] | d-spacing [Å] | Relative Intensity. [%] |
|---|---|---|---|
| 1 | 5.1 | 17.44 | 52 |
| 2 | 10.2 | 8.70 | 9 |
| 3 | 11.7 | 7.54 | 10 |
| 4 | 12.3 | 7.20 | 12 |
| 5 | 14.0 | 6.32 | 8 |
| 6 | 14.7 | 6.03 | 73 |
| 7 | 15.3 | 5.80 | 26 |
| 8 | 16.5 | 5.39 | 93 |
| 9 | 17.2 | 5.17 | 73 |
| 10 | 17.6 | 5.04 | 90 |
| 11 | 18.1 | 4.89 | 18 |
| 12 | 18.6 | 4.76 | 55 |
| 13 | 20.4 | 4.35 | 59 |
| 14 | 21.1 | 4.22 | 100 |
| 15 | 23.1 | 3.85 | 31 |
| 16 | 23.7 | 3.76 | 16 |
| 17 | 24.8 | 3.59 | 39 |
| 18 | 25.6 | 3.48 | 18 |
| 19 | 26.0 | 3.42 | 13 |
| 20 | 26.3 | 3.39 | 23 |
| 21 | 27.7 | 3.22 | 25 |
| 22 | 28.2 | 3.17 | 13 |
| 23 | 28.8 | 3.10 | 6 |
| 24 | 29.2 | 3.06 | 7 |
| 25 | 30.2 | 2.96 | 10 |

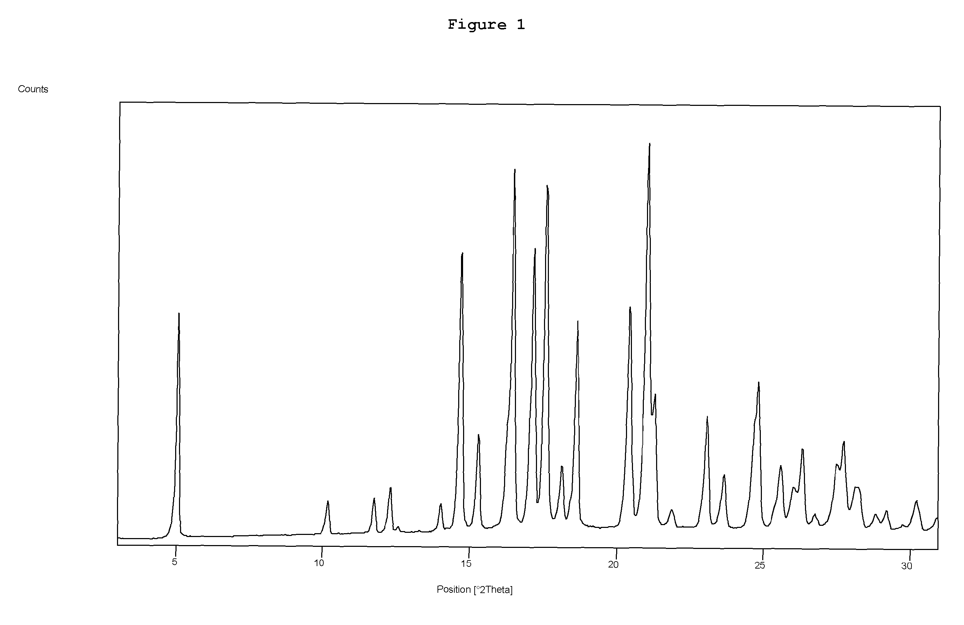

The term "d-spacing" means the interplanar spacing between successive atomic planes in the crystal; "Theta" (Θ) is the angle between the atomic plane and both the incident and reflected beams. The X-ray powder diffraction spectrum of Form (I) is shown in Figure 1.

All X-ray powder diffraction patterns reported herein were obtained by a Phillips PW3040/60 X'Pert PRO diffractometer using CuK_{α} radiation (Filter: Ni; Voltage: 45kV; Current: 40mA).

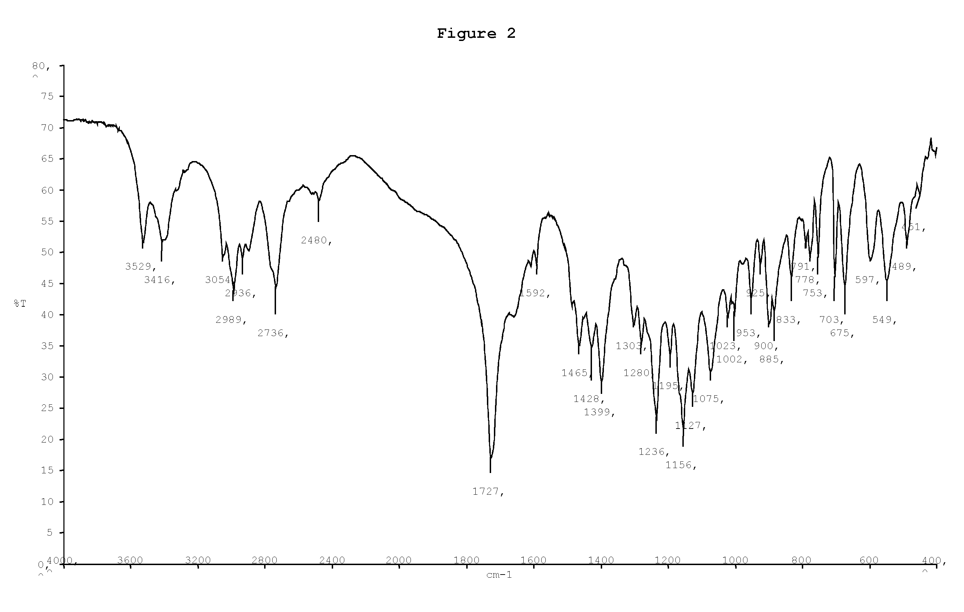

Rivastigmine hydrogentartrate of Form I is further characterized by its infrared absorption spectrum as shown in Figure 2.

All FT-IR spectra reported herein were recorded as KBr disc on a FT-IR spectrometer Spectrum 100 and System GX Perkin Elmer at a resolution of 4 cm⁻¹.

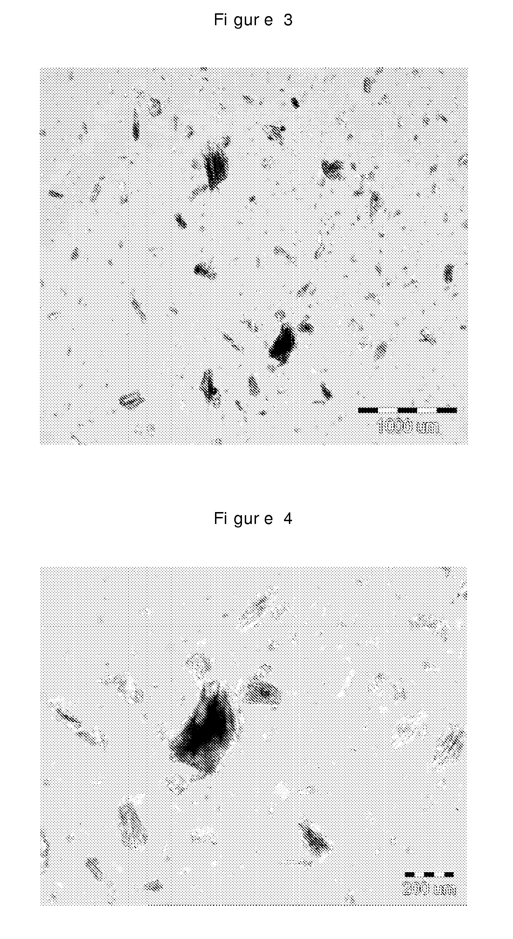

Figures 3 and 4 show microscopic pictures of crystals of rivastigmine hydrogentartrate of Form I.

Rivastigmine hydrogentartrate form I has a melting point of 125°C.

Rivastigmine hydrogentartrate of polymorphic form (II) is characterized by the following significant peaks in its X-ray powder diffraction pattern at 2Θ: 9.5, 11.3, 13.2, 14.1, 15.5, 19.1, 20.0° ±0.2.

The X-ray powder diffraction pattern of Form (II) comprises the following peaks:

**Table 2**

| No. | Diffraction angles [°; 2Θ] | d-spacing [Å] | Relative Intensity. [%] |
|---|---|---|---|
| 1 | 9.5 | 9.33 | 21 |
| 2 | 11.3 | 7.83 | 24 |
| 3 | 12.3 | 7.22 | 6 |
| 4 | 12.7 | 6.98 | 4 |
| 5 | 13.2 | 6.72 | 18 |
| 6 | 14.1 | 6.28 | 25 |
| 7 | 15.5 | 5.70 | 45 |
| 8 | 16.3 | 5.42 | 14 |
| 9 | 17.0 | 5.21 | 15 |
| 10 | 17.7 | 5.02 | 5 |
| 11 | 19.1 | 4.66 | 43 |
| 12 | 19.5 | 4.55 | 20 |
| 13 | 20.0 | 4.43 | 100 |
| 14 | 20.8 | 4.27 | 13 |
| 15 | 22.3 | 3.99 | 21 |
| 16 | 23.3 | 3.82 | 7 |
| 17 | 24.7 | 3.61 | 17 |
| 18 | 25.7 | 3.47 | 14 |
| 19 | 26.5 | 3.36 | 29 |
| 20 | 27.0 | 3.31 | 20 |
| 21 | 27.4 | 3.25 | 7 |
| 22 | 27.8 | 3.21 | 4 |
| 23 | 29.5 | 3.03 | 16 |
| 24 | 30.6 | 2.92 | 3 |

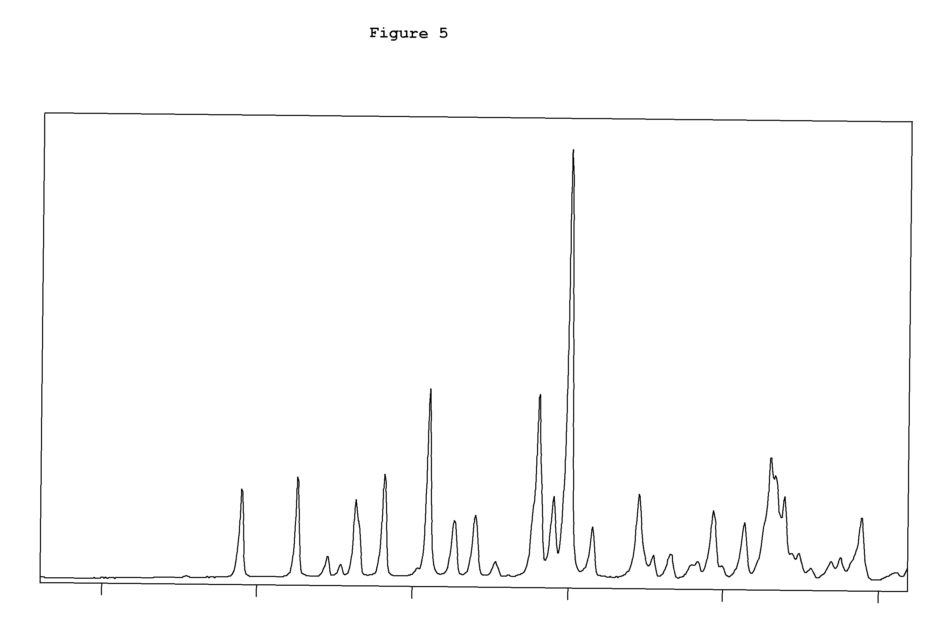

The X-ray powder diffraction spectrum of Form (II) is shown in Figure 5.

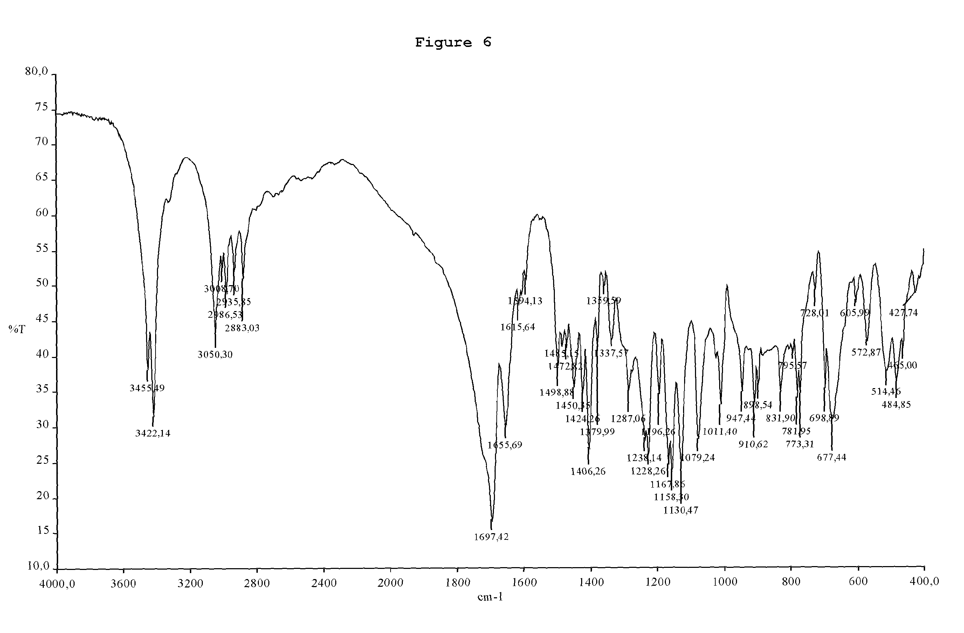

Rivastigmine hydrogentartrate of Form II is further characterized by its infrared absorption spectrum as shown in Figure 6.

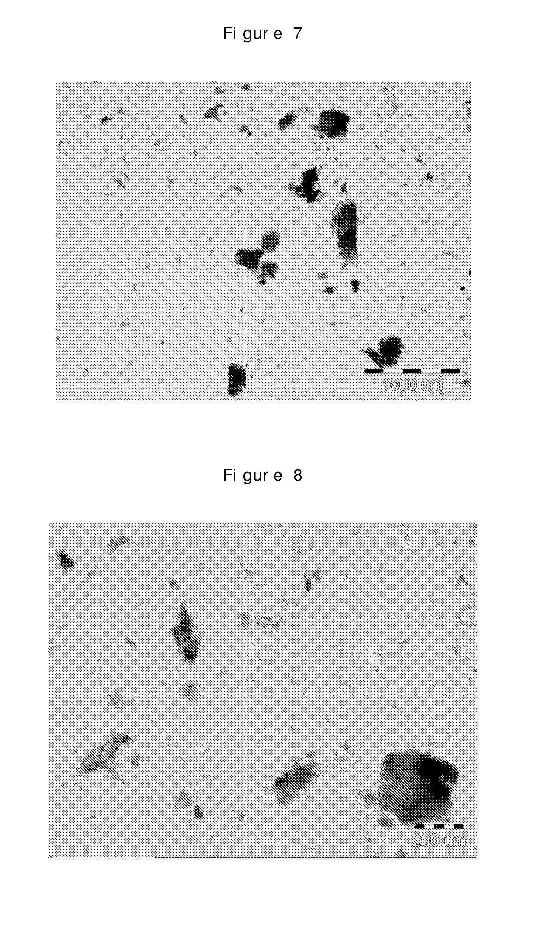

Figures 7 and 8 show microscopic pictures of crystals of rivastigmine hydrogentartrate of Form II.

Rivastigmine hydrogentartrate form II has a melting point of 100°C.

The new polymorphic forms of rivastigmine hydrogentartrate of the present invention are characterized by a different thermal behavior. The thermal behavior can be measured in the laboratory by known techniques such as capillary melting point, thermogravimetric analysis (TGA) and differential scanning calorimetry (DSC), and can be used to distinguish the novel polymorphic forms from others.

It is known that the working-up and processing properties of polymorphic forms of a pharmaceutically active ingredient such as filterability, drying properties, solubility, compressibility can differ significantly. Polymorphs I and in particular polymorph II of the present invention have uniform, well-defined morphologies and they are morphologically and therapeutically stable which is advantageous from the point of reproducibility. Furthermore, they have an advantageous dissolution rate, good bioavailability, they show little inter-individual variation, and are chemical stable. They can be easily processed by filtration, drying, and compression into tablets.

The polymorphic forms of rivastigmine hydrogentartrate of the present invention are particularly suitable for preparing medicaments. They can be used to improve the performance characteristics of pharmaceutical products. Furthermore, they enlarge the repertoire of materials that a formulation scientist has available for designing pharmaceutical dosage forms, such as dosage forms with a targeted release profile or other desired characteristics. In addition, the polymorphic forms of the present invention can easily be converted to an amorphous form. The skilled person now has the possibility to choose from different forms of rivastigmine hydrogentartrate with different physical properties. Having the knowledge that the polymorphic forms exist, the skilled person is in a position to control and optimize the process of formulation according to physical properties of solid state forms, in order to obtain a final dosage form comprising desired solid state form of rivastigmine hydrogentartrate. Also, having the knowledge that the polymorphic forms exist the skilled person is in a position to avoid incorporation of uncontrolled mixtures of different solid state forms into the pharmaceutical formulation.

Rivastigmine base can advantageously be prepared by reacting 3-(1-dimethylamino ethyl)phenol or a salt thereof with sodium hydride and ethyl(methyl)carbamic chloride as outlined in the examples. Preferably 3-(1-dimethylamino ethyl)phenol or a salt thereof is first dissolved in a solvent and then sodium hydride is added, followed by the addition of ethyl(methyl)carbamic chloride. 3-(1-Dimethylamino ethyl)-phenol or the salt thereof can be in racemic or preferably in optically active(S)-3-(1-dimethylamino ethyl) phenol form.

Alternatively, any other reaction sequence yielding rivastigmine base can be used, for instance the syntheses outlined in GB 2 203 040, WO 03/101917, WO 2004/037771, WO 2005/058804, WO 2006/068386, or CN1486973.

If racemic 3-(1-dimethylamino ethyl)phenol or a racemic salt thereof is used, the obtained rivastigmine base may be further treated with di-p-toluoyl-D-tartaric acid monohydrate in order to obtain the optically pure di-p-toluoyl tartrate salt of (S)-3-(1-(dimethylamino)ethyl)phenyl ethyl(methyl)carbamate which can be further converted to optically pure rivastigmine hydrogentartrate.

If optically active(S)-3-(1-dimethylamino ethyl) phenol or an optically active salt thereof is reacted with sodium hydride and ethyl(methyl)carbamic chloride, optically active rivastigmine base is obtained which can be further converted to rivastigmine hydrogentartrate.

It is preferred to use 1 to 1.5 mole, preferably about 1.2 mole of sodium hydride per mole of 3-(1-dimethylamino ethyl)phenol or its salt. Ethyl(methyl)carbamic chloride is preferably used in an amount of 1 to 2 mole, preferably about 1.5 mole per mole of 3-(1-dimethylamino ethyl)phenol or its salt.

The base can be converted to rivastigmine hydrogentartrate by methods known to the skilled person, e.g. by reacting rivastigmine base with hydrogentartrate in a solvent Preferred solvents for the above process are lower alcohols, e.g. C₁₋₃-alkohols, preferably methanol, ethanol or isopropyl alcohol, methyl ethyl ketone, acetonitrile and acetone.

Racemic 3-(1-(dimethylamino)ethyl)phenyl ethyl(methyl)-carbamate, (S)-3-(1-(dimethylamino)ethyl)phenyl ethyl(methyl)-carbamate, racemic rivastigmine hydrogentartrate, optically active rivastigmine hydrogentartrate and mixtures thereof can be purified with chiral columns for enantiomeric purification comprising chiral selectors or stationary phases; batch and/or supercritical-fluid chromatography can be used.

Preferred stationary phases are polysacharide-derived chiral stationary phases, such as cellulose tris (3,5-dimethylphenylcarbamate) immobilized on a silica support. The mobile phase can be selected from hexane and alcohols. Alcohols and in particular ethanol are preferred. Optionally additives such as diethyl amine, triethyl amine or trifluor acetic acid can be added in an amount of 0.01 to 0.5 vol. %, preferrably 0.05 to 0.01 vol. %.

Another preferred group of stationary phases are Pirkle type chiral stationary phases such as π-electron donor/π-electron acceptors. In this instance the mobile phase can be selected from hexane, alcohol and water. Preffered alcohols are ethanol and/or isopropyl alcohol. Optionally additives such as diethyl amine, triethyl amine or triflour acetic acid can be added in an amount of 0.01 to 0.5 vol. %, preferrably 0.05 to 0.01 vol. %.

Rivastigmine hydrogentartrate of polymorphic form I or II according to the present invention is prepared by a process which comprises the following steps:
a) crystallization of rivastigmine hydrogentartrate;
b) aging the crystals obtained in step (a);
c) drying the crystals of step (b).

For the preparation of rivastigmine hydrogentartrate of polymorphic form I and II according to the present invention optically pure rivastigmine hydrogentartrate is preferably used as a starting material, i.e. rivastigmine hydrogentartrate having an optical purity within the range of 99 to 100 %, preferably higher than 99.5 % and most preferably higher than 99.8 %. The different polymorphic forms of rivastigmine hydrogentartrate can be obtained by crystallization from different solvents, different cooling and/or aging conditions.

According to a first variant of the process of the present invention, crystallization step (a) of rivastigmine hydrogentartrate is achieved by providing a solution of rivastigmine hydrogentartrate in a solvent at a first temperature T1 in order to obtain a crystallization mixture, and crystallizing the product from the crystallization mixture by decreasing the temperature from T1 to a second temperature T2. Rivastigmine hydrogentartrate or, alternatively, tartaric acid and rivastigmine base can be used to prepare the solution of rivastigmine hydrogentartrate.

In order to obtain a clear solution the temperature of the initial suspension can be raised up to reflux temperature of the solvent. The hot solution is then cooled. Before cooling the solution is preferably filtered. Optionally, the solution may be concentrated by evaporation of solvent, preferably under vacuum, during crystallization.

The method of the present invention is characterized in that cooling is performed according to a defined temperature profile. For preparing rivastigmine hydrogentartrate of Form I, the temperature is decreased from the first temperature T1 to the second temperature T2 within the period of time 1 hour to 24 hours, preferably 2 to 12 hours. T1 is preferably about the reflux temperature of the solvent and T2 is preferably within the range of -20 °C to 20 °C, more preferable -10 °C to 10 °C.

The crystals which form upon cooling are then subjected to aging step (b). Preferably the mixture of step (a) comprising the crystals is kept at a temperature within the above range specified for T2 for 0.01 to 15 hours, more preferably for 6 to 12 hours, and the crystals are then isolated e.g. by filtration. The crystals are preferably dried under reduced pressure of less than 100 mbar to obtain rivastigmine hydrogentartrate of Form I. The drying temperature is preferably 32°C to 40 °C.

Preferred solvents for the above process are lower alcohols, e.g. C₁₋₃-alkohols, preferably ethanol or isopropyl alcohol, methyl ethyl ketone, acetonitrile and acetone.

For preparing rivastigmine hydrogentartrate of Form II the temperature is decreased from the first temperature T1 to the second temperature T2 within the period of time 1 hour to 300 hours, preferably 24 to 50 hours. T1 is preferably about the reflux temperature of the solvent and T2 is preferably within the range of -20 °C to 40 °C, preferable 0 °C to 30 °C.

The crystals which form upon cooling are preferably kept at a temperature within the above range for 20 hours to 300 hours, more preferably for 20 to 50 hours, and are then isolated e.g. by filtration. The crystals are preferably dried under reduced pressure of 150 to 800 mbar to obtain rivastigmine hydrogentartrate of Form II. The drying temperatures is preferably 20 °C to 30 °C.

Preferred solvents for preparing Form II are lower alcohols, e.g. C₁₋₃-alkohols, preferably ethanol, acetonitrile and acetone.

Once crystals of Form I or Form II have been prepared, these crystals can be used as seeding material in future processes. The seeding material is preferably added during cooling of the solution in order to initiate nucleation and to shorten the aging time to obtain rivastigmine hydrogentartrate of Form I or II.

For preparing rivastigmine hydrogentartrate of Form II by use of a seeding material the temperature of the crystallization mixture is preferably first decreased from a first temperature T1 to the second temperature T2' At T2' the crystallization mixture is preferably inoculated with the seeding material and then the mixture is either kept at the temperature T2' or optionally cooled to the temperature T2.

T1 is preferably about the reflux temperature of the solvent and T2' is preferably 10 to 40 °C below the reflux temperature of the solvent. T2 is preferably in the range of 0 °C to 40 °C, more preferable 5 °C to 30 °C. The mixture is optionally kept at the final temperature T2' or T2 for 6 hours to 300 hours, more preferably for 20 to 50 hours. Then the crystals are isolated and preferably dried under reduced pressure as described above.

According to another variant of the process of the present invention, crystallization of rivastigmine hydrogentartrate is induced by the addition of an anti-solvent to a solution of rivastigmine hydrogentartrate in a solvent. A clear solution of rivastigmine hydrogentartrate is prepared as described above. Optionally the solution may be concentrated by evaporation of solvent under vacuum. Preferred solvents for forming form I are lower alcohols, e.g. C₁₋₃-alkohols, preferably methanol, isopropyl alcohol or ethanol. Preferred solvents for forming form II are lower alcohols, e.g. C₁₋₃-alkohols, preferably ethanol, acetonitrile and acetone.

Next, the anti-solvent is added to the solution. Preferably anti-solvent having a temperature of -20°C to 50°C, preferable 0°C to 25°C, is added to the solution of rivastigmine hydrogentartrate that is preferably kept at a temperature of 0°C to 30°C, preferable 10°C to 30°C. The anti-solvent is preferably added slowly, i.e. over a period of 1 hour to 24 hours. During the addition of the anti-solvent the mixture is preferably stirred.

Preferred anti-solvents are ethers, esters, ketones and hydrocarbons. Preferred ethers are isopropylmethyl ether and tert-butylmethyl ether; a preferred ester is ethyl acetate. Preferred ketones are diethyl ketone, isopropyl methyl ketone and methyl i-butyl ketone. Preferred hydrocarbons are hexane, heptane and octane.

The anti-solvent is preferably added to the solution of rivastigmine hydrogentartrate in a volume ratio of solvent to anti-solvent from 1 : 10 to 1 : 50, preferably 1 : 15 to 1 : 25

Alternatively, the solution of the rivastigmine hydrogentartrate can be added to the anti-solvent. In this instance the temperature of the solution is preferably kept at 0°C to 40°C, more preferably 15°C to 25°C during the addition of the anti-solvent. The anti-solvent preferably has a temperature of -20°C to 50°C, preferable -10°C to 10°C. The solution is preferably added gradually, e.g. drop-wise to the anti-solvent. During the addition the mixture is advantageously stirred.

Prior to mixing the solution of rivastigmine hydrogentartrate and anti-solvent, the anti-solvent can be inoculated with crystals of the desired rivastigmine hydrogentartrate polymorphic form.

The crystals which form upon addition of the anti-solvent are aged, isolated and dried as described above for Form I or Form II depending on which crystalline form is desired.

The crystals are first subjected to aging step (b). For forming Form I the mixture of step (a) comprising the crystals is preferably kept at a temperature within the above range specified for T2 for 0.01 to 15 hours, more preferably for 6 to 12 hours, and crystals are then isolated e.g. by filtration. The crystals are preferably dried under reduced pressure of less than 100 mbar. The drying temperature is preferably 32°C to 40 °C.

For forming Form II the crystals which form upon addition of the anti-solvent are preferably kept at a temperature within the above range for 20 hours to 300 hours, more preferably for 20 to 50 hours, and are then isolated e.g. by filtration. The crystals are preferably dried under reduced pressure of 150 to 800 mbar to obtain rivastigmine hydrogentartrate of Form II. The drying temperatures is preferably 20 °C to 30 °C.

According to another embodiment rivastigmine hydrogentartrate of Form I is prepared by spray-drying a solution comprising rivastigmine hydrogentartrate. A clear solution of rivastigmine hydrogentartrate can be prepared as described above and this solution is then subjected to spray drying. The preferred solvent for spray-drying is ethanol. The drying gas can be air or another inert gas such as nitrogen, argon and carbon dioxide. Air is preferred. The temperature of the inlet gas is preferably within the range of 80 to 120°C, preferably 90 to 110 °C.

Rivastigmine hydrogentartrate of Form I can also be prepared by lyophilization which is preferably followed by further drying of the obtained product. Preferably a solution of rivastigmine hydrogentartrate in water, methanol, ethanol, acetonitrile, acetone or a mixture thereof is used for freeze drying. Further drying of the lyophilized product is performed under reduced pressure of less than 100 mbar to obtain rivastigmine hydrogentartrate of Form I. The drying temperature is preferably 32°C to 40 °C.

The rivastigmine hydrogentartrate of the present invention preferably has an average particle size in the range of 5 to 300 µm, preferably 20 to 150 µm, more preferably 50 to 100 µm. The term "average particle size" or "particle size" as used herein refers to the volume mean diameter of particles.

The average particle size is determined with a Malvern Mastersizer. The particles to be subjected to the particle size measurement are first suspended in isoparaffin oil (e.g. Isopar®) and then subjected to size determination in a Malvern Mastersizer MS 2000 instrument. Usually, 100-800 mg of substance are dispersed in 5-8 ml of oil. According to manufacturer's information, the Malvern Mastersizers allows for a measurement of particle size distributions within the range of 20 nm to 2000 µm, with a precision of better than 0.5%. Particle sizes are determined by measuring the angular distribution of laser light scattered by a homogeneous suspension of particles. The size distribution is determined from the light scattering data using the theory of light scattering developed by Gustav Mie.

The processes of the present invention surprisingly result in the formation of polymorphically pure rivastigmine hydrogentartrate. Preferably, the rivastigmine hydrogentartrate of Form I and of Form II according to the present invention is free of other polymorphic or amorphous forms of rivastigmine hydrogentartrate. Form I shown in Figure 1 is free of polymorphic Form II and other polymorphic or amorphous forms; and Form II shown in Figure 5 is free of polymorphic Form I and other polymorphic or amorphous forms. By "free of other polymorphic forms" it is meant that 90 to 100 % by weight, preferably at least 95 %, most preferably at least 99,8 % of the product have the desired polymorphic form.

Amorphous rivastigmine hydrogentartrate and rivastigmine hydrogentartrate of Form I or Form II according to the present invention is therapeutically stable and is particularly suitable for preparing medicaments, in particular for the treatment of Alzheimer's disease.

The pharmaceutical compositions may be in a solid or liquid dosage form. Exemplary solid dosage forms include tablets, capsules, sachets, lozenges, powders, pills, pellets, or granules. The solid dosage form may be, for example, a immediate release dosage form, a fast melt dosage form, orally disintegrating dosage form, modified release dosage form, lyophilized dosage form, delayed release dosage form, extended release dosage form, prolonged release dosage form, pulsatile dosage form, mixed immediate and modified release dosage form, or a combination thereof. Solid dosage forms are preferred. More preferably, the solid dosage form is an immediate release dosage form offering advantages regarding the bioavailability of the active compound.

Pharmaceutical dosage forms comprising rivastigmine hydrogentartrate can be prepared by a process comprising the steps of mixing rivastigmine hydrogentartrate according to the present invention with at least one pharmaceutically acceptable excipient and forming the mixture into a pharmaceutical dosage form. Rivastigmine hydrogentartrate and the one or more excipients can be mixed in the presence or in the absence of solvent.

The manufacturing process for oral solid capsules can be a conventional process comprising the steps of preparing particles, drying, final blending and/or capsule filling. These particles are typically in the form of granules or pellets.

The active ingredient can be mixed with excipients for preparing granules and then be subjected to granulation, or alternatively granules can be formed without the active ingredient and these granules and optional further ingredients are then mixed with the active ingredient in the final blending step to form e.g. a capsule filling mixture. Granules can be produced by common granulation techniques , such as wet granulation or dry granulation methods.

The process conditions for preparing pharmaceutical dosage forms can chosen such that the solid state form of rivastigmine hydrogentartrate remains unchanged or such that the solid state form is changed during the preparation of the dosage form. For instance, use of a solvent during granulation of a mixture comprising the active ingredient favors conversion of polymorphic form I to the amorphous form while dry granulation does not result in a change of the crystalline form. Similarly, separate granulation of the excipients without the active ingredient in the presence or absence of a solvent, followed by combining these granules with rivastigmine hydrogentartrate in the absence of solvent does not cause a change of the solid state form.

Suitable excipients for preparing granules are preferably selected from the following excipients:
Fillers comprising lactose in anhydrous or hydrated form such as lactose monohydrate, microcrystalline cellulose, siliconized microcrystalline cellulose, starch, powdered cellulose, sugar derivatives such as mannitol, sorbitol, lactitol, saccharose, earth alkali phosphates such as calcium phosphate, calcium carbonate, calcium lactate and mixtures thereof;
binders comprising polyvinyl pyrolidone, cellulose ethers such as hydroxypropyl cellulose, hydroxypropylmethyl cellulose, hydroxyethyl cellulose, microcrystalline cellulose, starch, pregelatinised starch, polymethacrylate and mixtures thereof;
disintegrants comprising starch derivatives such as corn starch and pregelatinised starch, low substituded hydroxypropyl cellulose, microcrystalline cellulose, carboxymethyl cellulose salts such as calcium and sodium salts, polacrillin, potasium, crospovidone, sodium starch glycolate and mixtures thereof;
lubricants such as sodium stearyl fumarate, metal stearates such as magnesium, calcium, aluminium stearate, hydrogenated oils such as hydrogenated vegetable or castor oil, talc, colloidal silicon dioxide and mixtures thereof,
and surfactants.

Surfactant molecules can be classified based on nature of the hydrophilic group within the molecule. According to the present invention the following types of surfactants can be used:

Anionic surfactants, wherein the hydrophilic group carries a negative charge, such as carbonyl (RCOO⁻), sulphonate (RSO₃⁻) or sulfate (ROSO₃⁻). Preferred anionic surfactants are potassium laurate, CH₃(CH₂)₁₀COO⁻K⁺, and sodium lauryl sulfate, CH₃(CH₂)₁₁SO₄⁻Na⁺.

Cationic surfactants, wherein the hydrophilic group carries a positive charge (e.g., quaternary ammonium halides, R₄N⁺Cl⁻) . Preferred cationic surfactants are cetrimide, mixtures comprising mainly trimethyltetradecylammonium bromide and smaller amounts of dodecyltrimethylammonium bromide and hexadecyltrimethylammonium bromide, benzalkonium chloride, mixtures of alkylbenzyldimethylammonium chlorides of the general formula [C₆H₅CH₂N⁺(CH₃)₂R]Cl⁻, wherein R represents a mixture of alkyl residues from C₈H₁₇ to C₁₈H₃₇.

Ampholytic surfactants (also called zwitterionic surfactants), wherein a molecule contains, or can potentially contain, both a negative and a positive charge, (e.g., the sulfobetaines, RN⁺(CH₃)₂CH₂CH₂SO₃⁻). Preferred ampholytic surfactants are N-dodecyl-*N,N*-dimethylbetaine, C₁₂H₂₅N⁺(CH₃)₂CH₂COO⁻.

Nonionic surfactants, wherein the hydrophilic group carries no charge but derives its water solubility from highly polar groups such as hydroxyl or polyoxyethylene (OCH₂CH₂O-) groups. Preferred nonionic surfactants are polyoxyethylated glycol monoethers (e.g. cetomacrogol), sorbitan esters (Spans^{®}) and polysorbates (Tweens^{®}).

A special group of nonionic surfactants are poloxamers, that are defined as nonionic polyoxyethylene-polyoxypropylene copolymers. They are chemically similar in composition, differing only in the relative amounts of propylene and ethylene oxides added during manufacture.

Lubricants, glidants and antiadherents used in solid pharmaceutical compositions of the present invention, are herein not defined as surface-acting agents (surfactants).

Different types of mannitol can be used for preparing granules, such as spray-dried mannitol, mannitol for direct compression and conventional mannitol. The average particle size of the mannitol is preferably within the range of 10 to 500 µm, preferably 100 to 200 µm.

Different types of microcrystalline cellulose can be used for preparing granules. The average particle size of the microcrystalline cellulose is preferably within the range of 10 to 500 µm, preferably 20 to 150 µm. Suitable examples of such types of microcrystalline cellulose are Avicel PH 101 having an average particle size of 50 µm and Avicel PH 102 with an average particle size of 90 µm, as specified by the manufacturer, or other commercially available microcrystalline celluloses. Microcrystalline cellulose can be used as a diluent, binding, lubricating and disintegrating agent.

The granulation can be performed either by using solvents (wet granulation) or by dry granulation. Water or water miscible organic solvents can be used in the wet granulation step using state of the art granulation equipment such as fluid bed granulators, high or low shear mixers. The obtained granulate can be further dried by using microwave, fluid bed or convection dryers, drying chambers with or without using reduced pressure or vacuum. Dry granulation can be performed by using compactors or briquetting techniques. Preferred solvents for wet granulation are selected from water and/or alcohols.

Excipients to form a capsule filling mixture are preferably selected from lubricants such as sodium stearyl fumarate, metal stearates such as magnesium, calcium, aluminium stearate, hydrogenated oils such as hydrogenated vegetable or castor oil, talc, colloidal silicon dioxide and mixtures thereof.

The granules obtained according to above described methods can be encapsulated into hard gelatin capsules or capsules consisting of hydroxypropylmethyl cellulose.

As an alternative to the encapsulation of granules, capsules can be filled with the dry mixture, wherein dry mixture is prepared using conventional manufacturing processes comprising mixing of active ingredient and above excipients, followed by final blending and/or capsule filling.

Alternatively, a blended composition may be compressed directly into a compacted dosage form using direct compression techniques. Direct compression produces more uniform tablets without granules. The proper use of the above and other excipients in direct compression tabletting is known to those skilled in the art. The tablets produced by direct compression can be of different size and shape. In one embodiment the tablets can be in the form of microtablets with diameter 1.0 to 4.0 mm, preferably 1.5 to 3 mm, which can be used as such or which can be filled into capsules. In the latter case 1 to 40, preferably 2 to 30 microtablets are filled into one capsule. Different doses can be achieved by varying the number of microtablets within the capsules.

Rivastigmine hydrogentartrate of Form I or Form II according to the present process can advantageously also be used for the preparation of orally disintegrating tablets.

Orally disintegrating tablets can be produced according the process comprising the steps of preparing granules containing the active ingredient, mixing the granules excipients, and compressing the obtained mixture to the desired form.

In an alternative embodiment of the present invention orally disintegrating tablets can be produced according the process comprising the following steps: preparing granules, mixing the granules with active ingredient and excipients and compressing the obtained mixture to the desired form.

During the preparation of the granules and in particular during the mixing or spraying step further excipients can be added, such as filler, preferably lactose or starch, and/or binder, such as gelatin, polyvinyl pyrrolidone, starch, pregelatinized starch, cellulose derivatives, such as hydroxypropylmethyl cellulose (HPMC), hydroxypropyl cellulose (HPC), ethylcellulose and carboxymethyl cellulose (CMC) or their salts. The HPC is preferably low-substituted hydroxypropyl cellulose, i.e. hydroxypropyl cellulose with a content of hydroxypropyl groups of 7.0 to 12.9 % by weight.

The active ingredient can be mixed with excipients for preparing granules and then be subjected to granulation, or alternatively granules can be formed without the active ingredient and these granules and optional further ingredients are then mixed with the active ingredient to form orally disintegrating formulations.

The granulate comprising the active ingredient can be dried (the loss on drying is preferably less than 2 %, most preferably less than 1.5 %) and subsequently mixed with other excipients. The mixture is then compressed to tablets at < 60 N.

Alternatively, granulate not containing active ingredient can be dried (the loss on drying is preferably less than 2 %, most preferably less than 1.5 %) and subsequently mixed with excipients and the active ingredient, and the mixture is then compressed to tablets at < 60 N.

Examples of suitable excipients for the preparation of tablets are calcium silicate, magnesium stearate, sodium stearyl fumarate, Pharmaburst® (SPI Pharma), sweeteners and flavors.

The calcium silicate can be used in crystalline form, amorphous form or as a mixture thereof. The average particle size of calcium silicate is preferably in the range of 1 to 500 µm, more preferably 1 to 100 µm.

Aspartame is a preferred sweetener. Other examples of sweeteners are acesulfame K, sucralose, alitame, saccharin sodium, dipotassium glycyrrhizinate, and thaumatin.

Limette flavor is a preferred flavor.

Various types of punches can be used for compressing tablets such as flat, biconvex, bevel edged, round, oval or capsule shaped punches and punches having other forms. The friability of the tablets is preferably less than 1 %, determined according to the method described in Ph. Eur. 07/2005:0478; Chapter 2.9.7.

The disintegration times of the obtained tablets were below 30 s. They were measured in purified water according to the method described in Ph. Eur. 07/2005:0478; Chapter 2.9.1. Test A.

If a prolonged release of the drug is desired, controlled release polymers such as swellable cellulose ethers, e.g. hydroxypropylmethyl cellulose or hydroxypropyl cellulose which exhibit a viscosity of 100 mPas or more at room temperature in 2% w/w dispersion in water, ethylcellulose, methacrylic ester copolymers, ammonium methacrylate copolymers, natural saccharides such as xanthan gum, alginic acid salts, carragenans or mixtures thereof are incorporated into the tablet cores.

For prolonged release of the drug solid dosage forms such as matrix tablets or coated granules or pellets with controlled release properties can be used. Matrix tablets are based on homogenous mixture of drug and controlled release polymer and at least one pharmaceutically acceptable excipient i.e. binders, fillers and lubricants, as previously described. Matrix tablets can be produced by direct compression of the mixture of ingredients or via granulation and compression of produced granulate together with extragranular excipients such as lubricants into tablets. Alternatively prolonged release coated pellets or granules can be produced. In one of the embodiment of the invention pellet core are produced by direct pelletization of mixture of drug and excipients selected from binders, fillers, and optionally controlled release polymers such as by extrusion and spheronization or alternative processes of direct pelletization such as pelletization in high shear mixer or tangential spray fluid bed equipment. Alternatively controlled release pellets can be produced by coating of inactive core such as sugar spheres made of sucrose and starch and (or) microcrystalline cellulose with a suspension or solution containing drug, binder (see above) and optionally further excipients selected from surfactants, fillers, antitacking agents such as talc, earth alkaly stearates or partial glycerides such as glycerol monostearate. Water or organic solvents such as ethanol or mixture thereof can be used for preparation of suspensions or solutions. Obtained pellets are further coated with controlled release coating containing water insoluble polymers such as ethylcellulose, derivatives of polymethacrylates and other acceptable excipients selected from plasticizers, antitacking agents and optionally water soluble excipients selected from saccharides such as lactose, sucrose, mannitol and (or) soluble polymers such as low viscosity types of hydroxypropyl methyl cellulose (viscosity of 2% solution in water at 25°C < 100 mPas), hydroxyethyl cellulose, povidone. Pellets can be additionally dried after each coating phase, in the same equipment where the coating phase took place or in drying chambers. Curing phase can be optionally performed on final pellets by keeping the pellets at elevated temperatures such as 30 to 60°C, preferably 35 to 50°C, most preferably 40 to 45°C. Prolonged release mean that less than 50% of drug is released after 2 hours (using the paddle method according to Ph. Eur., phosphate buffer pH 6.8), 40 to 70% after 6 hours and more than 80% after 12 hours.

### Description of the Figures

Figure 1 is X-ray powder diffraction pattern of rivastigmine hydrogentartrate Form I.
Figure 2 is a FT-IR spectrum of rivastigmine hydrogentartrate Form I.
Figure 3 is microscopic picture of crystals of rivastigmine hydrogentartrate Form I.
Figure 4 is microscopic picture of crystals of rivastigmine hydrogentartrate Form I at a different magnification.
Figure 5 is X-ray powder diffraction pattern of rivastigmine hydrogentartrate Form II.
Figure 6 is a FT-IR spectrum of rivastigmine hydrogentartrate Form II.
Figure 7 is microscopic picture of crystals of rivastigmine hydrogentartrate Form II.
Figure 8 is microscopic picture of crystals of rivastigmine hydrogentartrate Form II at a different magnification.
Figure 9 shows X-ray powder diffraction patterns of rivastigmine hydrogentartrate Form I (curve a), of a pharmaceutical formulation comprising rivastigmine hydrogentartrate Form I (curve b), of a placebo formulation comprising no rivastigmine hydrogentartrate Form I (curve c) and of a formulation comprising amorphous rivastigmine hydrogentartrate (curve d).

In the following the invention will be described by use of examples. In the examples, high resolution HPLC is used to determine the purity of rivastigmine hydrogentartrate. The tests are carried out in XTerra RP-18 (250 × 4,6 mm, 5 µm particles) column (as described in J. of Pharma and Biomed. Analysis Volume 37, Issue 1 , 7 February 2005, Pages 57-63). The mobile phase is a gradient of 0,01M sodium heptanesulphonate, pH 3,0 and acetonitrile. The chromatograph is equipped with a UV detector set at 217 nm, flow rate is 0.8 ml/min at 40°C.

High resolution HPLC is also used to determine the enantiomeric purity of rivastigmine hydrogentartrate. The tests are carried out in CHIRALCEL OD-H 250x4,6mm column. The mobile phase is HEXAN : ethanol : TFA : DEA = 84 : 16 : 0,1 : 0,1. The chromatograph is equipped with a UV detector set at 210 nm, flow rate is 0.5 ml/min at 40°C.

### Examples

### Example 1:

### (i) Synthesis of rivastigmine base

### a) Preparation of 3-(1-dimethylaminoethyl)phenol

An amount of 10.10 g (50.0 mmol) of 3-(1-dimethylaminoethyl)-phenol hydrochloride was dissolved in 75 ml of water and 100 ml of dichloromethane. 50 ml of 1 M NaOH were added dropwise to the mixture while stirring. The pH was adjusted to 9.7 with 1 M NaOH and the reaction mixture was stirred for another 20 minutes. The organic layer was separated and 50 ml of brine were added thereto and stirred for 20 minutes. The organic layer was separated, dried over Na₂SO₄ (7.0 g) and filtered. The organic layer was concentrated under reduced pressure to obtain 7.48 g of an oily residue of 3-(1-dimethylaminoethyl)phenol which rapidly crystallized.

### b) Preparation of 3-(1-(dimethylamino)ethyl)phenyl ethyl-(methyl) carbamate (rivastigmine base)

An amount of 5.25 g (30.0 mmol) of 3-(1-dimethylaminoethyl)-phenol was dissolved in 70 ml of acetonitrile. To the solution was slowly added 1.30 g (32.5 mmol) of sodium hydride and stirred for 30 minutes. 6.20 g (51 mmol) of ethyl(methyl)-carbamic chloride were slowly added to the solution. The reaction mixture was stirred for 20 hours. The mixture was filtered and concentrated. 100 ml of water were added and the pH was adjusted with 0.1 M NaOH to pH 12, followed by extraction with ether (3x100 ml). The organic layer was then washed with 100 ml of brine, dried over sodium sulfate, filtered and concentrated. 6.20 g of an oily residue of rivastigmine base were obtained.

### (ii) Synthesis of rivastigmine base (alternative)

An amount of 6.06 g (30.0 mmol; 99.3 area %) of 3-(1-dimethylaminoethyl)phenol hydrochloride was dissolved in 45 ml of water and stirred for 10 minutes. 60 ml of dichloromethane was added and stirred for additional 10 minutes. 30 ml of 1 M NaOH was added dropwise in period of 30 minutes and the pH was set to 9.7 with 1 M NaOH. The reaction mixture was stirred for another 30 minutes. The layers were separated. The organic layer was dried with 2 g of Na₂SO₄, and filtered. After the filtration 30 ml of acetonitrile were added and concentrated at 600 mbar and a temperature of 50°C twice. After the evaporation of the solvent, the pressure was slowly reduced to 350 mbar and maintained to complete the evaporation of dichloromethane. 3-(1-dimethylaminoethyl)phenol was obtained as a solution in acetonitrile.

An amount of 1.1 g (27.5 mmol, 60 %, 1.05 mole) of sodium hydride suspended in 15 ml of acetonitrile was added to 1.0 mole of the above obtained 3-(1-dimethylaminoethyl)phenol (as acetonitrile solution) within a period of 30 minutes while maintaining the reaction mixture at the room temperature.

After the addition of sodium hydride an amount of 3.1 ml (3.44 g, 97 % 27.5 mmol, 1.05 molar equivalent) of ethyl(methyl)-carbamic chloride were added within a period of 30 minutes.

The reaction mixture was stirred for 2 hours at room temperature and then the solvent was removed under vacuum at 50 mbar and 50°C. 55 ml of ether and 35 ml of water were added to the residue, the pH was set to 11 with 0.1 M NaOH and stirred for 20 minutes. The layers were separated. The water phase was washed with 25 ml of ether and the organic phases were combined, dried with 2.0 g of Na₂SO₄ and filtered. The filtrate was concentrated to obtain 6.4 g of an oily substance of 3-(1-(dimethylamino)ethyl)phenyl ethyl(methyl)carbamate. Yield from 1-(3-hydroxyphenyl)-N,N-dimethyl-ethanaminium chloride was 85 %, purity of the product 97.7 area %.

### (iii) Synthesis of rivastigmine hydrogentartrate

An amount of 1.30 g (5.2 mmol) of 3-(1-(dimethylamino)ethyl)-phenyl ethyl(methyl)carbamate as obtained in step (i) and 2.10 g (5.2 mmol) of (+)-di-p-toluoyl-D-tartaric acid monohydrate were dissolved in 13 ml of methanol/water (2:1) solution under heating. The reaction mixture was refluxed for 1 hour and cooled to room temperature. The precipitate was filtered and four times recrystallized in a minimal volume of methanol/water solution to obtain optically pure di-p-toluoyl tartrate salt of (S)-3-(1-(dimethylamino)ethyl)phenyl ethyl-(methyl)carbamate. The salt was dissolved in 1 M NaOH and extracted into ether to obtain an optically pure oily residue of (S)-3-(1-(dimethylamino)ethyl)phenyl ethyl(methyl)carbamate which was dissolved in ethanol, mixed with hydrogentartrate, refluxed and cooled to room temperature.

### Preparation of polymorphic forms

### Example 2: Preparation of rivastigmine hydrogentartrate of polymorphic form II

Rivastigmine hydrogentartrate as obtained in Example 1 was dissolved in absolute ethanol (concentration 1 g / 7 ml) at ~70 °C. The solution was allowed to cool to 25 °C, then it was inoculated with rivastigmine hydrogentartrate crystals of form II. The suspension was allowed to crystallize at 20 °C for 14 hours. The product was filtered and dried under the vacuum at 25 °C. Yield 0,65 g. Crystals having the X-ray diffraction pattern shown in Figure 5 were obtained.

### Example 3: Preparation of rivastigmine hydrogentartrate of polymorphic form II

An amount of 200 mg of rivastigmine hydrogentartrate was dissolved in 2 ml of acetonitrile at reflux temperature. The clear solution was allowed to cool to 30 °C in about 2 hours. The clear solution was then inoculated with 2 mg of rivastigmine hydrogentartrate form II. During the crystallization the suspension was slowly cooled to 25 °C and stirred for 14 hours. The product was filtered under nitrogen flow and dried in vacuum at 25 °C. Yield: 0. 85 g. Crystals having the X-ray diffraction pattern shown in Figure 5 were obtained.

### Example 4: Preparation of rivastigmine hydrogentartrate of polymorphic form II

An amount of 2 g of rivastigmine hydrogentartrate was dissolved in 30 ml of acetonitrile at reflux temperature. While stirring with a magnetic stirrer the solution was cooled to room temperature in about 2 hours. The solution became turbid at room temperature. The suspension was allowed to crystallize for 48 hours. a very dense slurry was isolated and dried in vacuum at 25 °C. Yield: 1.77 g, Crystals having the X-ray diffraction pattern shown in Figure 5 were obtained. The compound had a melting point of 100°C.

### Example 5: Preparation of rivastigmine hydrogentartrate of polymorphic form I

An amount of 1 g of rivastigmine hydrogentartrate was dissolved in 25 ml of acetone at reflux temperature. The solution was allowed to cool to 25 °C. During cooling the mixture was rigorously stirred. The suspension was additionally cooled to 15 °C in 1 hour and filtered under nitrogen. The product was dried under vacuum at 25 °C. Yield: 0.83 g. Crystals having the X-ray diffraction pattern shown in Figure 1 were obtained. The compound had a melting point of 125°C.

### Example 6: Preparation of rivastigmine hydrogentartrate of polymorphic form I

An amount of 1 g of rivastigmine hydrogentartrate was dissolved in 5 ml of ethanol at ~70 °C. The solution was cooled to -10 °C where crystallization started. The suspension was allowed to crystallize at -10 °C for 14 hours. The obtained slurry was filtered and dried at 25 °C under vacuum. Yield: 0,69 g. Crystals having the X-ray diffraction pattern shown in Figure 1 were obtained.

### Example 7: Preparation of rivastigmine hydrogentartrate of polymorphic form I

An amount of 1 g of rivastigmine hydrogentartrate was dissolved in 1 ml of methanol. A clear solution was obtained at room temperature. A few drops of methanol solution were added to 20 ml diethyl ketone while stirring at room temperature. The still clear solution was cooled to -10°C. Rivastigmine hydrogentartrate spontaneously nucleated in diethyl ketone. The rest of the methanol solution of rivastigmine hydrogentartrate was added drop wise in about 3 hours. During the addition of methanol suspension stirring was continued and the temperature was maintained below 0°C. The suspension was additionally stirred for 3 hours and was then allowed to warm to room temperature. The product was filtered under dry nitrogen, and dried in a vacuum dryer for 3 hours at 40 °C. Yield: 0.85g. Crystals having the X-ray diffraction pattern shown in Figure 1 were obtained.

### Example 8: Preparation of rivastigmine hydrogentartrate of polymorphic form I

An amount of 1 g of rivastigmine hydrogentartrate was dissolved in 1 ml of methanol. A clear solution was obtained at room temperature. While stirring, 20 ml of diisopropyl ether were added. The suspension was stirred, cooled to -10 °C and allowed to warm to 0 °C in 3 hours. The product was filtered under dry nitrogen, and dried in a vacuum dryer for 3 hours at 40 °C. Yield: 0.87 g. Crystals having the X-ray diffraction pattern shown in Figure 1 were obtained.

### Example 9: Preparation of rivastigmine hydrogentartrate of polymorphic form I

An amount of 20 g of rivastigmine hydrogentartrate was dissolved in 200 ml of ethanol and subjected to spray drying. The temperature of the input air was kept between 90 and 110°C. Crystals having the X-ray diffraction pattern shown in Figure 1 were obtained.

### Preparation of pharmaceutical compositions

### Example 10: Capsules containing rivastigmine hydrogentartrate in amorphous form

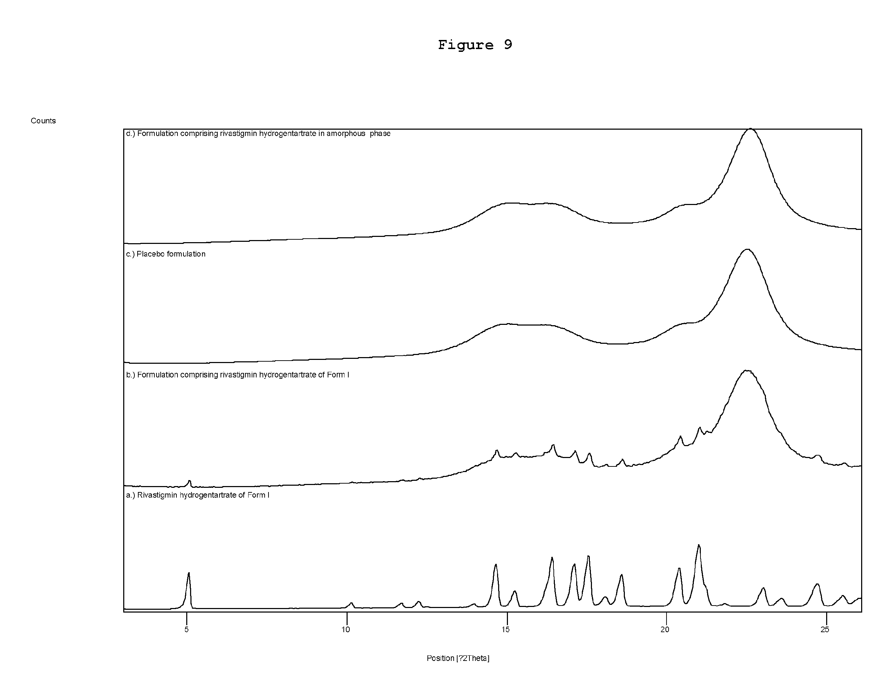

2.4 g of rivastigmine hydrogentartrate (Form I), 155.6 g of microcrystalline cellulose and 1.2 g of hydroxypropylmethylcellulose were homogenized in a high-shear mixer and then 100 g of purified water were sprayed onto the mixture. The granulate obtained was dried in fluid-bed dryer at an inlet air temperature of 60°C. The loss on drying (measurement performed in a Mettler Toledo HR73 halogen moisture analyzer at 85°C for 20 minutes) of the sieved granulate was 1.6% by weight. 0.7 g of anhydrous colloidal silica were added to the granulate and mixed. Finally, 0.1 g of magnesium stearate were added to obtain an encapsulating mixture. The loss on drying (measurement performed in a Mettler Toledo HR73 halogen moisture analyzer at 85°C for 20 minutes) of the mixture was 1.7% by weight. The formulation had the X-ray powder diffraction pattern shown in Figure 9 (curve d). The formulation comprises rivastigmine hydrogentartrate in amorphous form.

The encapsulating mixture obtained as described above was filled into hard gelatin capsules (capsule size »3«). The total weight of 10 filled capsules was in the range of 0.203-0.211 g. The theoretical weight of rivastigmine in each capsule was 1.5 mg.

In stress stability testing, initially (t=0) the total amount of impurities was 0.02%? (analyzed by HPLC). After 1 month on 50°C (in closed container), total impurities raised to 0.03%, and on 50°C/75% relative humidity (RH) (in opened container) to 0.27%.

### Example 11: Capsules containing rivastigmine hydrogentartrate in amorphous form

9.6 g of rivastigmine hydrogentartrate (Form I), 144.8 g of microcrystalline cellulose and 4.8 g of hydroxypropylmethylcellulose were homogenized in high-shear mixer and then 115 g of purified water were sprayed onto this mixture. The granulate obtained was dried in fluid-bed dryer at an inlet air temperature of 60°C. The loss on drying (measurement performed in a Mettler Toledo HR73 halogen moisture analyzer at 85°C for 20 minutes) of the sieved granulate was 1.5% by weight. 0.7 g of anhydrous colloidal silica were added to granulate and mixed. Finally, 0.1 g of magnesium stearate were added to obtain an encapsulating mixture. The loss on drying (measurement performed in a Mettler Toledo HR73 halogen moisture analyzer at 85°C for 20 minutes) of the mixture was 1.3% by weight.

The encapsulating mixture obtained as described above was filled into hard gelatin capsules (capsule size »3«). The total weight of 10 filled capsules was in the range of 0.202-0.211 g. The theoretical weight of rivastigmine in each capsule was 6 mg.

### Example 12: Capsules containing rivastigmine hydrogentartrate form I

155.6 g of microcrystalline cellulose and 1.2 g of hydroxypropylmethylcellulose were homogenized in a high-shear mixer. 120 g of purified water were sprayed onto the mixture of excipients as listed above. The granulate obtained was dried a in fluid-bed dryer at an inlet air temperature of 65°C. The loss on drying (measurement performed in a Mettler Toledo HR73 halogen moisture analyzer at 85°C for 20 minutes) of the sieved granulate was 2.0% by weight. 2.4 g of rivastigmine hydrogentartrate (Form I) and 0.7 g of anhydrous colloidal silica were added to the granulate and mixed. Finally, 0.1 g of magnesium stearate were added to obtain an encapsulating mixture. The loss on drying (measurement performed in a Mettler Toledo HR73 halogen moisture analyzer at 85°C for 20 minutes) of the mixture was 2.0% by weight.

The encapsulating mixture was then filled into hard gelatin capsules (capsule size »4«). The total weight of 10 filled capsules was in the range of 0.194-0.202 g. The theoretical weight of rivastigmine in each capsule was 1.5 mg. The formulation had the X-ray powder diffraction pattern shown in Figure 9 (curve b). The formulation comprises rivastigmine hydrogentartrate Form I.

In stress stability testing, initially (t=0) the total amount of impurities was 0.02% (analyzed by HPLC). After 1 month at 50°C (in closed container), total impurities remain unchanged (0.02%), and on 50°C/75% RH (in opened container) raised to 0.25%.

### Example 13: Capsules containing rivastigmine hydrogentartrate form I

144.8 g of microcrystalline cellulose and 4.8 g of hydroxypropylmethylcellulose were homogenized in a high-shear mixer, then 100 g of purified water were sprayed onto the mixture. The granulate obtained was dried in fluid-bed dryer at an inlet air temperature of 65°C. The loss on drying (measurement performed in a Mettler Toledo HR73 halogen moisture analyzer at 85°C for 20 minutes) of the sieved granulate was 2.7% by weight. 9.6 g of rivastigmine hydrogentartrate form I and 0.7 g of anhydrous colloidal silica were added to the granulate and mixed. Finally, 0.1 g of magnesium stearate were added to obtain an encapsulating mixture. The loss on drying (measurement performed in a Mettler Toledo HR73 halogen moisture analyzer at 85°C for 20 minutes) of the mixture was 2.5% by weight.

The encapsulating mixture was filled into hard gelatin capsules (capsule size »4«). The total weight of 10 filled capsules was in the range of 0.194-0.202 g. The theoretical weight of rivastigmine in each capsule was 6 mg.

### Example 14: Capsules containing rivastigmine hydrogentartrate form I

2.4 g of rivastigmine hydrogentartrate form I, 155.6 g of microcrystalline cellulose, 1.2 g of hydroxypropylmethylcellulose and 0.7 g of anhydrous colloidal silica were homogenized in a high-shear mixer. 0.1 g of magnesium stearate were added to obtain an encapsulating mixture. The loss on drying (measurement performed in a Mettler Toledo HR73 halogen moisture analyzer at 85°C for 20 minutes) of the mixture was 3.7% by weight.

The encapsulating mixture was filled into hard gelatin capsules (capsule size »2«). The total weight of 10 filled capsules was in the range of 0.221-0.229 g. The theoretical weight of rivastigmine in each capsule was 1.5 mg.

In stress stability testing, initially (t=0) the total amount of impurities was 0.02% (analyzed by HPLC). After 1 month at 50°C, total impurities raised to 0.03%, and at 50°C/75% relative humidity (RH) to 0.23%.

### Example 15: Tablets containing rivastigmine hydrogentartrate in amorphous form

2.4 g of rivastigmine hydrogentartrate form I, 86.6 g of mannitol, 10 g of microcrystalline cellulose and 10 g of low-substituted hydroxypropylcellulose LH-21 (ShinEtsu; the hydroxypropoxyl content was 10.0 to 12.9%; >90% of the particles are smaller than 75 µm) were homogenized in high-shear mixer. Next, 50 g of purified water were sprayed onto the mixture. The granulate obtained was dried in fluid-bed dryer at an inlet air temperature of 60°C. The loss on drying (measurement performed in a Mettler Toledo HR73 halogen moisture analyzer at 85°C for 20 minutes) of the sieved granulate was 0.7% by weight. 1 g of aspartame, 5 g of limette flavor, 12 g of cross-linked povidone and 20 g of calcium silicate were added to the granulate and mixed. Finally, 3 g of magnesium stearate were added to obtain a compressing mixture.

The compressing mixture was compressed into tablets (round punches with diameter 7 mm) with a theoretical weight of 150 mg. The hardness of the tablets (n=20) was within the range of 19-28 N. The disintegration time of the tablets (determined according to Ph.Eur. monograph »Disintegration of tablets and capsules«) in purified water at 37°C was 10 seconds. The theoretical weight of rivastigmine in each tablet was 1.5 mg.

In stress stability testing, initially (t=0) the total amount of impurities was 0.10% (analyzed by HPLC). After 1 month at 50°C (in closed container), total impurities raised to 0.74%, and at 50°C/75% relative humidity (RH) (in opened container) to 2.16%. A direct comparison with the stability of the capsules described in Examples 8 and 9 is not possible because fast dissolving tablets are more temperature and moisture sensitive than capsules.

### Example 16: Tablets containing rivastigmine hydrogentartrate form I

86.6 g of mannitol, 10 g of microcrystalline cellulose and 10 g of low-substituted hydroxypropylcellulose LH-21 (ShinEtsu) were homogenized in a high-shear mixer. Next, 40 g of purified water were sprayed onto the mixture. The granulate obtained was dried in fluid-bed dryer at an inlet air temperature of 60°C. The loss on drying (measurement performed in a Mettler Toledo HR73 halogen moisture analyzer at 85°C for 20 minutes) of the sieved granulate was 0.7% by weight. 2.4 g of rivastigmine hydrogentartrate form I, 1 g of aspartame, 5 g of limette flavor, 12 g of cross-linked povidone and 20 g of calcium silicate were added to the granulate and mixed. Finally, 3 g of magnesium stearate were added to obtain a compressing mixture.

The compressing mixture was compressed into tablets (round punches with diameter 7 mm) with a theoretical weight of 150 mg. The hardness of the tablets (n=20) was in the range of 16-28 N. The disintegration time of the tablets (determined according to Ph.Eur. monograph »Disintegration of tablets and capsules«) in purified water at 37°C was 10 seconds. The theoretical weight of rivastigmine in each tablet was 1.5 mg.

### Example 17: Tablets containing rivastigmine hydrogentartrate in amorphous form

2.4 g of rivastigmine hydrogentartrate (Form I), 20 g of lactose monohydrate and 1 g of ethylcellulose were homogenized in a high-shear mixer. 4 g of ethanol (96 per cent) were sprayed onto this mixture. The granulate obtained was dried in a fluid-bed dryer at an inlet air temperature of 60°C. The loss on drying (measurement performed in a Mettler Toledo HR73 halogen moisture analyzer at 85°C for 20 minutes) of the sieved granulate was 0.5% by weight. 117.6 g of Pharmaburst™ type C1 (commercially available quick dissolve excipient/delivery system having a particle size within the range of 80 to 150 µm), 1 g of aspartame, and 5 g of limette flavor were added to the granulate and mixed. Finally, 3 g of sodium stearyl fumarate were added to obtain a compressing mixture.

The compressing mixture was compressed into tablets (round punches with diameter 7 mm) with a theoretical weight of 150 mg. The hardness of the tablets (n=20) was in the range of 23-32 N. The disintegration time of the tablets (determined according to Ph.Eur. monograph »Disintegration of tablets and capsules«) in purified water at 37°C was 12 seconds. The theoretical weight of rivastigmine in each tablet was 1.5 mg.

In stress stability testing, initially (t=0) the total amount of impurities was 0.04% (analyzed by HPLC). After 1 month at 50°C (in closed container), total impurities raised to 1.48%, and at 50°C/75% relative humidity (RH) (in opened container) to 1.67%. A direct comparison with the stability of the capsules described in Examples 8 and 9 is not possible because fast dissolving tablets are more temperature and moisture sensitive than capsules.

### Example 18: Tablets containing rivastigmine hydrogentartrate in amorphous form

The composition of the tablets was the same as described in Example 16, with the exception that sodium stearyl fumarate was substituted with the same amount (3 g) of magnesium stearate.

The compressing mixture was compressed into tablets (round punches with diameter 7 mm) with a theoretical weight of 150 mg. The hardness of the tablets (n=20) was within the range of 17-26 N. The disintegration time of the tablets (determined according to Ph.Eur. monograph »Disintegration of tablets and capsules«) in purified water at 37°C was 10 seconds. The theoretical weight of rivastigmine in each tablet was 1.5 mg.

## Claims

1. Rivastigmine hydrogentartrate in amorphous form or in a crystalline form **characterized by** an X-ray powder diffraction pattern exhibiting peaks at 2Θ values of 5.1, 14.7, 16.5, 17.6, 18.6, 20.4 and 21.1° ±0.2° (Form I), or 2Θ values of 9.5, 11.3, 13.2, 14.1, 15.5, 19.1, 20.0° ±0.2° (Form II).

2. Rivastigmine hydrogentartrate of Form I according to claim 1 specified by an X-ray powder diffraction pattern exhibiting peaks at the following diffraction angles:
| Diffraction angles [°; 2Θ] | Relative Intensity. [%] |
|---|---|
| 5.1 | 52 |
| 10.2 | 9 |
| 11.7 | 10 |
| 12.3 | 12 |
| 14.0 | 8 |
| 14.7 | 73 |
| 15.3 | 26 |
| 16.5 | 93 |
| 17.2 | 73 |
| 17.6 | 90 |
| 18.1 | 18 |
| 18.6 | 55 |
| 20.4 | 59 |
| 21.1 | 100 |
| 23.1 | 31 |
| 23.7 | 16 |
| 24.8 | 39 |
| 25.6 | 18 |
| 26.0 | 13 |
| 26.3 | 23 |
| 27.7 | 25 |
| 28.2 | 13 |
| 28.8 | 6 |
| 29.2 | 7 |
| 30.2 | 10 |

3. Rivastigmine hydrogentartrate of Form I according to claim 1 or 2, having an infrared absorption spectrum as shown in Figure 2.

4. Rivastigmine hydrogentartrate of Form II according to claim 1 specified by an X-ray powder diffraction pattern exhibiting peaks at the following diffraction angles:
| Diffraction angles [°; 2Θ] | Relative Intensity. [%] |
|---|---|
| 9.5 | 21 |
| 11.3 | 24 |
| 12.3 | 6 |
| 12.7 | 4 |
| 13.2 | 18 |
| 14.1 | 25 |
| 15.5 | 45 |
| 16.3 | 14 |
| 17.0 | 15 |
| 17.7 | 5 |
| 19.1 | 43 |
| 19.5 | 20 |
| 20.0 | 100 |
| 20.8 | 13 |
| 22.3 | 21 |
| 23.3 | 7 |
| 24.7 | 17 |
| 25.7 | 14 |
| 26.5 | 29 |
| 27.0 | 20 |
| 27.4 | 7 |
| 27.8 | 4 |
| 29.5 | 16 |
| 30.6 | 3 |

5. Rivastigmine hydrogentartrate of Form II according to claim 1 or 4, having an infrared absorption spectrum as shown in Figure 6.

6. Rivastigmine hydrogentartrate according to any one of claims 1 to 5, having a particle size of 5 to 300 µm.

7. A process for producing rivastigmine hydrogentartrate according to any one of claim 1 to 6, which comprises the steps:
a) crystallization of rivastigmine hydrogentartrate;
b) aging the crystals obtained in step (a);
c) drying the crystals of step (b).

8. The process of claim 7, wherein crystallization in step (a) is achieved by providing a solution of rivastigmine hydrogentartrate in a solvent at a first temperature T1 in order to obtain a crystallization mixture, and crystallizing the product from the crystallization mixture by decreasing the temperature from T1 to a second temperature T2.

9. The process of claim 8 for producing rivastigmine form (I), wherein the first temperature T1 is about the reflux temperature of the solvent and the second temperature T2 is within the range of -20°C to 20°C.

10. The process of claim 9, wherein the temperature is decreased from T1 to T2 within a time period of 1 hour to 24 hours.

11. The process of claim 9 or 10, wherein the crystals which form upon cooling are maintained at T2 for 0.01 to 15 hours.

12. The process of any one of claims 9 to 11, wherein drying in step (c) is performed under reduced pressure of 100 mbar or less.

13. The process of any one of claims 9 to 12, wherein drying in step (c) is performed at a temperature of 32°C to 40 °C.

14. The process of any one of claims 9 to 13, wherein the solvent is selected from the group of lower alcohols, C₁₋₃-alkohols, ethanol, isopropyl alcohol, methyl ethyl ketone, acetonitrile and acetone.

15. The process of claim 8 for producing rivastigmine form (II), wherein the first temperature T1 is about the reflux temperature of the solvent and the second temperature T2 is within the range of -20°C to 40°C.

16. The process of claim 15, wherein the temperature is decreased from T1 to T2 within a time period of 1 hour to 300 hours.

17. The process of claim 15 or 16, wherein the crystals which form upon cooling are maintained at T2 for 20 to 300 hours.

18. The process of any one of claims 15 to 17, wherein drying in step (c) is performed under reduced pressure of 150 mbar to 800 mbar.

19. The process of any one of claims 15 to 18, wherein drying in step (c) is performed at a temperature of 20°C to 30 °C.

20. The process of any one of claims 15 to 19, wherein the solvent is selected from the group of lower alcohols, C₁₋₃-alkohols, ethanol, acetonitrile and acetone.

21. The process of claim 7, wherein crystallization in step (a) is induced by the addition of an anti-solvent to a solution of rivastigmine hydrogentartrate in a solvent.

22. The process of claim 7, wherein crystallization in step (a) is induced by the addition of a solution of rivastigmine hydrogentartrate in a solvent to an anti-solvent.

23. The process of claim 21 or 22 for producing rivastigmine form (I), wherein the solvent is selected from lower alcohols, C₁₋₃-alkohols, methanol, ethanol, and isopropyl alcohol.

24. The process of claim 23 for producing rivastigmine form (II), wherein the solvent is selected from lower alcohols, C₁₋₃-alkohols, ethanol, acetonitrile and acetone.

25. The process of any one of claim 21 to 24, wherein the anti-solvent is selected from ethers, esters, ketones and hydrocarbons.

26. The process of any one of claim 7 to 25, wherein rivastigmine hydrogentartrate seed crystals of Form I or Form II are added.

27. A process for producing rivastigmine hydrogentartrate form I comprising spray drying a solution of rivastigmine hydrogentartrate.

28. A process for producing rivastigmine hydrogentartrate form I comprising lyophilizing a solution of rivastigmine hydrogentartrate.

29. Pharmaceutical formulation comprising rivastigmine hydrogentartrate according to any one of claim 1 to 6.

30. Process for producing a pharmaceutical formulation comprising rivastigmine hydrogentartrate, comprising the steps of mixing rivastigmine hydrogentartrate according to any one of claims 1 to 6 with at least one pharmaceutically acceptable excipient and forming the mixture into a pharmaceutical dosage form.

31. The process of claim 30, wherein the rivastigmine hydrogentartrate and the one or more excipients are mixed in the presence of solvent.

32. The process of claim 30, wherein the rivastigmine hydrogentartrate and the one or more excipients are mixed in the absence of solvent.

33. The process of any one of claims 30 to 32, wherein the mixture is granulated.

34. The process of any one of claims 30 to 33, wherein the one ore more excipients are first granulated and then mixed with the rivastigmine hydrogentartrate.
